Europäisches Patentamt

(19) European Patent Office

Office européen des brevets

(11) Publication number: **0 276 982**
**A2**

## (12) EUROPEAN PATENT APPLICATION

(21) Application number: 88300630.6

(22) Date of filing: 26.01.88

(51) Int. Cl.⁴: **C 12 P 7/64**
//(C12P7/64,C12R1:645)

(30) Priority: 27.01.87 JP 15108/87

(43) Date of publication of application:
03.08.88 Bulletin 88/31

(84) Designated Contracting States:
AT BE CH DE ES FR GB GR IT LI LU NL SE

(71) Applicant: Suntory Limited
1-40 Dojimahama 2-chome Kita-ku
Osaka-shi Osaka-fu 530 (JP)

(72) Inventor: Shinmen, Yoshifumi
S-304, 8-1, Enmyojitoriimae Oyamazaki-cho
Otokuni-gun Kyoto (JP)

Yamada, Hideaki
19-1, Matsugasakikinomoto-cho Sakyo-ku
Kyoto-shi Kyoto (JP)

Shimizu, Sakayu
14, Nishinokyohakuraku-cho Nakagyo-ku
Kyoto-shi Kyoto (JP)

(74) Representative: Ford, Michael Frederick et al
MEWBURN ELLIS & CO. 2/3 Cursitor Street
London EC4A 1BQ (GB)

(54) Process for production of fatty acids having high degree of unsaturation.

(57) A process for the production of a mixture of highly unsaturated fatty acids comprising the steps of (1) culturing a microorganism belonging to the genus Mortierella and capable of synthesizing highly unsaturated fatty acids, (2) preparing intact cells or products derived from the cells having an ability to synthesize highly unsaturated fatty acids from a precursor thereof, (3) converting the precursor to the highly unsaturated fatty acids in the presence of the intact cells or a product derived from the cells to obtain a reaction mixturecontaining the highly unsaturated fatty acids, and (4) recovering a mixture of the highly unsaturated fatty acids from the reaction mixture; and a process for the production of a highly unsaturated fatty acid comprising the steps of (1) culturing a microorganism belonging to the genus Mortierella and capable of synthesizing highly unsaturated fatty acids, (2) preparing intact cells or products derived from the cells having an ability to synthesize highly unsaturated fatty acids from a precursor thereof, (3) converting the precursor to the highly unsaturated fatty acids in the presence of the intact cells or a product derived from the cells to obtain a reaction mixture, (4) recovering a mixture of the highly unsaturated fatty acids from the reaction mixture containing the highly unsaturated fatty acids, and (5) isolating an individual highly unsaturated fatty acid.

**Description**

PROCESS FOR PRODUCTION OF FATTY ACIDS HAVING HIGH DEGREE OF UNSATURATION

The present invention relates to a process for production of a fatty acid having a high degree of unsaturation and a lipid containing such unsaturated fatty acids.

Processes for production of fatty acids having a high degree of unsaturation and using a microorganism belonging to the genus Aspergillus, Rhodotorula or Fusarium are known from Japanese Examined Patent Publications Nos. 56-19231, 56-19232 and 56-19233. Further, Japanese Unexamined Patent Publication No. 60-168391 discloses a process for the production of γ-linolenic acid using a microorganism belonging to the genus Mortierella. However, a process for the production of fatty acids having a high degree of unsaturation (hereinafter, highly unsaturated fatty acids) by an enzyme reaction using a microorganism belonging to the genus Mortierella is not known.

Accordingly, the present invention provides new processes for the production of highly unsaturated fatty acids and a lipid comprising highly unsaturated fatty acids by an enzyme reaction using a microorganism belonging to the genus Mortierella.

More specifically the present invention provides a process for the production of a mixture of highly unsaturated fatty acids comprising the steps of:

(1) culturing a microorganism belonging to the genus Mortierella and capable of synthesizing highly unsaturated fatty acids;

(2) preparing intact cells or products derived from cells having the ability to synthesize highly unsaturated fatty acids from a precursor thereof;

(3) converting the precursor to highly unsaturated fatty acids in the presence of intact cells or the product derived from the cells to obtain a reaction mixture; and

(4) recovering a mixture of the highly unsaturated fatty acids from the reaction mixture.

The present invention also provides a process for the production of a highly unsaturated fatty acid comprising the steps of:

(1) culturing a microorganism belonging to the genus Mortierella and capable of synthesizing highly unsaturated fatty acids;

(2) preparing intact cells or products derived from cells having the ability to synthesize highly unsaturated fatty acids from a precursor thereof;

(3) converting the precursor to the highly unsaturated fatty acids in the presence of the intact cells or the product derived from the cells to obtain a reaction mixture;

(4) recovering a mixture of the highly unsaturated fatty acids from the reaction mixture; and

(5) isolating an individual highly unsaturated fatty acid.

In the present invention, the term "highly unsaturated fatty acid" denotes a fatty acid containing at least three double bonds in a carbon chain and having preferably 18 to 22 carbon atoms. Such highly unsaturated fatty acids include, for example α-linolenic acid, γ-linolenic acid, bishomo-γ-linolenic acid, arachidonic acid, eicosapentaenoic acid, docosahexanoic acid.

In the present invention, as a producer microorganism, any strain belonging to the genus Mortierella capable of producing highly unsaturated fatty acids can be used. For example, Mortierella elongata IFO 8570, Mortierella alpina IFO 8568, Mortierella exigua IFO 8571, Mortierella hygrophila IFO 5941, and Mortierella parvispora IFO 8574 can be used. These strains are stored in the Osaka Institute for Fermentation; 17-85, Juso-honmachi 2-chome, Yodogawa-ku, Osaka 532, Japan, and are available to the public without limitation.

Moreover, a new strain, Mortierella elongata SAM 0219, can be used. This strain was newly isolated from soil and identified by the present inventors, and was deposited with the Fermentation Research Institute, Agency of Industrial Science and Technology (FRI), Higashi 1-1-3, Tsukuba-shi, Ibaraki-ken, Japan as FERM P-8703 on March 19, 1986, and transferred to International Deposition under the Budapest Treaty as FERM BP-1239 on December 22, 1986.

The above-mentioned new strain SAM 0219 (FERM BP-1239) has the following taxonomical properties:

Cultural characteristics on various culture media
Culture condition: 25°C in the dark

1. Malt extract agar medium
Colonies grow fast, attaining a diameter of 28 to 31 mm in two days and a diameter of 65 to 72 mm in five days; colonies are lobed; the formation of aerial mycelium is scanty; sporulation is good; sporangiophores arise from the aerial hyphae; the mycelium has a garlic-like odour.

2. Potato dextrose agar medium
Colonies grow fast, attaining a diameter of 27 to 31 mm in two days and a diameter of 75 to 80 mm in five days; colonies form a rosette pattern of dense lobes; much aerial mycelium is formed at the center of the colony; the reverse side of the colony is yellowish white or yellow in colour; sporulation is poor; the mycelium has a rather strong garlic-like odour.

3. Czapek's agar medium

Colonies grow moderately fast, attaining a diameter of 22 to 24 mm in two days and a diameter of 50 to 53 mm in five days; the formation of aerial mycelium is scanty; occasionally, the aerial hyphae cling tightly to each other; sporulation is abundant; the mycelium has a garlic-like odour.

4. LCA agar medium (prepared according to Koichiro Miura and Mitsuyo Y. Kudo, "An agar-medium for aquatic Hyphomycetes" Transactions of the Mycological Society of Japan vol. 11, p 116 - 118, 1970)

Colonies grow fast, attaining a diameter of 27 - 29 mm in two days and a diameter of 64 to 66 mm in five days; colonies are lobed; the formation of aerial mycelium is scanty, except at the center of the colony; sporulation is good; sporangiophores arise from the aerial hyphae; the mycelium has a garlic-like odour.

Microscopic Examination

Sporangiophore, mode of branching sporangiophore, sporangium, sporangiospore, etc., were microscopically observed for microscopic preparates and the colony per se. from various media.

A sporangiophore is tapered and has a length of 87.5 to 320 μm, a width of 3 to 7.5 μm at the root, and a width of 1.0 to 2.5 μm at the top, and often branches at the root. A sporangium is spherical in form, has a diameter of 15 to 30 μm, contains many ascospores therein, and has an unclear color after the detaching of the sporangiospore. A sporangiospore is elliptical or, rarely, renal in form, has a smooth surface, and a size of 7.5 to 12.5 × 5 to 7.5 μm. A relatively large number of chlamydospores are formed. Chlamydosphores are present separately or, rarely, linked in a chain form. Occasionally, several mycelia appear from the edge of the chlamydosphore. The chlamydosphore is elliptical or subspherical in form, and has a size of 12.5 to 30 × 7.5 to 15 μm(?), or a diameter of 12.5 to 15 μm. Zygospores are not observed.

Physiological Properties

Optical growth condition:
pH:     6 to 9,
Temperature:     20°C to 30°C;
Range for growth:
pH:     4 to 10,
Temperature:     5°C to 40°C.

On the basis of the above-mentioned taxonomical properties, and according to J.A. von Arx, "The Genera of Fungi Sporulating in Pure Culture" 3rd ed., J. Cramer, 1981; and K.H. Domsch, W. Gams and T.H. Anderson, "Compendium of Soil Fungi", Academic Press, 1980, the strain SAM-0219 of the present invention is considered to be a fungus belonging to the genus Mortierella, because a sporangium is formed at a top of a sporangiophore, the sporangium has no collumella, the sporangiospore has no appendage, and the mycelium has a garlic-like odour.

Therefore, the taxonomical properties of the strain of the present invention were compared with those of known species of the genus Mortierella according to W. Gams," A key to the species of Mortierella, Persoonia 9: p 381 - 391, 1977. As a result, because the colony is not velvety, the mycelium has a garlic-like odour, a sporangiophore has a length of 87.5 to 320 μm and branches at only its lower part and does not branch racemosely, and a sporangium contains many sporangiospore therein, the strain in question was considered to fall under the genus Mortierella, subgenus Mortierella, section Hygrophila. The section Hygrophila includes 22 species. According to the comparison of the present strain with these 22 species, the present strain is similar to Mortierella zychae, M. elongatula, and M. elongata.

Therefore, the strain of the present invention was compared with the above-mentioned three strains, referring to K.H. Domsch, W. Gams, and T.-H. Anderson, "Compendium of Soil Fungi", Academic Press, 1980; W. Gams, Some New or Noteworthy Species of Mortierella"; Persoonia 9: 111 - 140, 1976; G. Linnemann, "Mortierella Coemans 1863; H. Zyche and R. Siepmann, "Mucorales Eine Beschreibung Aller Gattungen und Arten dieser Pilzgruppe", p 155 - 241, J. Cramer, 1965. The present strain is clearly different from M. zychae in the length and width of the sporangiophore at the base, and the size of the sporangium. Moreover, the present strain is different from M. elongatula in the shape and size of the sporangiosphore. The present strain is different from M. elongata in that sporangiophore is rather shorter, the chlamydosphore is ellipsoidal or subglobose in form, chlamydospores are rarely linked to each other in a chain form, and a small number of radiating hyphae exist. However, the present inventors concluded that such differences between the present strain and M. elongata are not sufficient to distinguish the present strain from M. elongata, and thus identified the strain of the present invention as Mortierella elongata, and designated it as strain SAM 0219.

Spores, mycelia, or a preculture is used as an inoculum for culturing the present strains. The medium used may be a liquid or solid medium. A liquid medium contains as a carbon source, for example, glucose, fructose, xylose, saccharose, maltose, soluble starch, molasses, glycerol, or mannitol. Nitrogen sources include organic substances such as peptones, yeast extract, meat extract, casamino acid, corn steep liquor, and inorganic substances such as sodium nitrate, ammonium nitrate, ammonium sulfate, and the like. If necessary, inorganic salts such as phosphate salts, magnesium sulfate, ferrous sulfate and cupric sulfate, and vitamins may be included in a medium. The concentration of these components is selected so that such components do not adversely affect the growth of the microorganism used. Practically, the concentration of the carbon source is 0.1 to 30% by weight, preferably 1 to 10% by weight, reflective to the total weight of the medium. The concentration of the nitrogen source is 0.01 to 5% by weight, preferably 0.1 to 2% by weight, relative to the total weight of the medium.

To enhance the production of target fatty acids, in addition to the above-mentioned medium components, hydrocarbons, fatty acids or salts thereof, or fats, are preferably added to a medium in an amount of 0.01% to 20%. Hydrocarbons are preferably added to a medium at the start of culturing, and fatty acids or salts thereof, or fats, are preferably added at the start of and/or during culturing. When such an additive is used during culturing, it is added at one time, stepwise, or continuously.

the culturing temperature ranges 5°C to 40°C. A pH value of the medium is 4 to 10, preferably 6 to 9.

Culturing is preferably carried out with aeration and/or agitation, with shaking in a liquid medium, or with standing, and is usually carried out for 2 to 10 days.

When culturing is carried out on a solid medium, the solid medium is composed of wheat bran, chaff or rice bran supplemented with water in an amount of 50 to 100% by weight relative to the wheat bran, chaff or rice bran. If necessary, the medium is supplemented with a small amount of nitrogen source, inorganic salts, and/or minor nutrients. Culturing is carried out at a temperature of 5°C to 40°C, preferably 20°C to 30°C, for 3 to 14 days.

The thus-obtained microbial cells contain an enzyme system for the production of highly unsaturated fatty acids, and in accordance with the present invention, cultured cells or a product derived from the cultured cells are used to convert a precursor to highly unsaturated fatty acids. In such a process, "cultured cells" include cells which have been cultured and are still present in a cultured broth and cells which are separated from a cultured broth and optionally washed with an appropriate washing solution. The separation of cultured cells from the cultured broth is carried out according to a conventional procedure such as filtration or centrifugation. The "product derived from cultured cells" includes dried cells, cells treated with an organic solvent and dried, and disrupted cells, as well as immobilized products of the above-mentioned cells or cell products, in which an enzyme system necessary to convert a precursor to the target highly unsaturated fatty acids is contained. The dried cells can be prepared by, for example, lyophilization or drying under a reduced pressure. For lyophilization, cells separated as above-mentioned are resuspended in water or an appropriate buffer such as a phosphate buffer, Tris-HCl and the resulting cell suspension is frozen, and dried under a reduced pressure. When drying under a reduced pressure, cells separated as above-mentioned in the form of a pellet are placed in a dryer, and the cells are dried under a reduced pressure. Dried cells treated with an organic solvent are prepared, for example, by suspending a cell pellet prepared as above in an organic solvent which is miscible with water, for example, acetone, methanol, ethanol or the like, to eliminate water from the cells, and then drying the treated cells. Disrupted cells are prepared according to a conventional procedure used for the disruption of microbial cells. For example, separated cells are suspended in an appropriate buffer such as a 0.05 M phosphate buffer, and then the cell suspension is frozen and disrupted in a mortar with a pestle. Alternatively, the cell suspension is treated with a homogenizer, Dyno-mil, French press, or ultrasonicator to disrupt cells and obtain a cell homogenate. Moreover, the above-mentioned cells and various kinds of products derived from the cells can be immobilized according to a conventional enzyme immobilizing procedure or cell immobilizing procedure, such as the bound method, the cross-linkage method, the entrapped method, the adsorbed method or the like.

The precursors for highly unsaturated fatty acids are preferably fatty acids having 2 to 20 carbon atoms such as acetic acid, butylic acid, caproic acid, caprylic acid, caprynic acid, lauric acid, myristic acid, myristoleic acid, palmitic acid, palmitoleic acid, stearic acid, oleic acid, linoleic acid, linolenic acid, octadecatetraenoic acid, arachidic acid, eicosenoic acid, eicosadienoic acid, eicosatrienoic acid, eicosatetraenoic acid, and the like. These fatty acids are used in a free form, or as salts such as sodium, potassium or ammonium salt, or esters such as methyl ester or ethyl ester. Alternatively, the above-mentioned fatty acids can be used as a component of fats or oils, such as triglycerides.

The reaction for conversion of the above-mentioned precursor to the target highly unsaturated fatty acids in the presence of the above-mentioned cells or cell product is carried out in an appropriate aqueous reaction medium. The reaction medium is, for example, water or a buffer such as a phosphate buffer, Tris-HCl buffer or the like.

If desirable or necessary, an emulsion promotor, for example, a surfactant such as Tween or Triton, or bile powder may be added to the reaction medium in an amount of 0.001% to 1%.

Reaction is carried out at a pH value of 3 to 10, preferably 5 to 8, at a temperature of 5 to 40°C, preferably 10 to 30°C, for 30 minutes to 10 days, preferably 10 to 48 hours. The reaction is carried out under a stationary condition, or preferably, with agitation or shaking to accelerate contact of the precursor with the cells or cell product containing the enzyme system for conversion of the precursor into highly unsaturated fatty acids.

After the above-mentioned reaction, target highly unsaturated fatty acids are recovered from the reaction mixture. When intact or dried cells are used as an enzyme source, during the reaction, lipids containing target fatty acids are mainly intracellularly accumulated and therefore, target fatty acids are recovered from the cells by the following procedure. After the reaction, cells are collected from the reaction mixture by a conventional means such as filtration or centrifugation, the cells are washed with water, and preferably, the washed cells are dried. When products derived from cells are used as an enzyme source, the reaction mixture is usually dried.

Drying is carried out by, for example, lyophilization or air-drying. The dried product is treated with an organic solvent or a mixture thereof, preferably under a nitrogen stream, to extract a lipid containing target fatty acids. The organic solvent or mixture thereof is, for example, ethers such as ethyl ether, hydrocarbons such as hexane, alcohols such as methanol or ethanol, halo-hydrocarbons such as chloroform or dichloromethane, petroleum ether, as well as a mixture of chloroform, methanol and water, or a combination of methanol and petroleum ether alternately used. By distilling off the solvent, a lipid containing concentrated target fatty acids is obtained.

Alternatively, wet cells or there action mixture can be subjected to direct extraction. In such a case, a water-miscible solvent such as methanol or ethanol, or a water-miscible solvent comprising the water-miscible solvent and water or other organic solvent is used. The extraction procedure is the same as described for dried cells.

The lipid thus obtained contains target fatty acids in the form of a lipid compound such as fat. Although the target fatty acids can be isolated in the form of a free acids, they are preferably isolated in the form of an ester with a lower alcohol, for example, as methyl esters. By converting target fatty acids to such esters, each is easily separated from other lipid components, and from other target fatty acids and other undesirable fatty acids formed during culturing, such as palmitic acid, oleic acid, linoleic acid and the like, which are also esterified at the same time as the target fatty acids are esterified. To obtain methyl esters of the target fatty acids, for example, the lipid prepared as described above is treated with a 5 to 10% hydrochloric acid solution in absolute methanol or a 10 to 50% $BF_3$ solution in methanol for 1 to 24 hours at a room temperature.

The mixture thus obtained is extracted with an organic solvent such as hexane, ethyl ether or ethyl acetate, to recover methyl ester of the target fatty acids. Next, the extract is dried over anhydrous sodium sulfate, and the solvent is distilled under a reduced pressure to obtain a residue mainly comprising a fatty acid mixture. The mixture contains, in addition to methyl esters of the target highly unsaturated fatty acids, methyl palmitate, methyl stearate, methyl oleate and the like. From the mixture, methyl esters of highly unsaturated fatty acids are isolated by column chromatography, low temperature crystallization, a urea-adducting method, or a combination thereof.

The isolated methyl esters of the target fatty acids are then hydrolyzed with an alkali and extracted with an organic solvent such as ethyl ether, ethyl acetate, or the like to obtain the target fatty acids in a free form.

Alternatively, target fatty acids can be obtained, without conversion to methyl ester, by alkalolysis with, for example, 5% sodium hydroxide at a room temperature for 2 to 3 hours, followed by extraction of the fatty acids from the alkalolysis product and isolation of the target fatty acids.

In accordance with the present invention, both a mixture of highly unsaturated fatty acids and an individual fatty acid can be obtained. To produce an individual fatty acid, the above-mentioned mixture of methyl esters of highly unsaturated fatty acids or mixture of highly unsaturated fatty acids is subjected to a conventional separation procedure such as urea-adducting method, column chromotography, or the like.

Examples
The present invention will now be further illustrated by, but is by no means limited to, the following examples.

Example 1. (Production of highly unsaturated fatty acids)
50 ml of a medium containing 2% glucose and 1% yeast extract (pH 6.0) was prepared and charged into a 500 ml-volume Meyer(?) flask, and the whole was autoclaved for 20 minutes at 120°C. After cooling, Mortierella elongata SAM 0219 (FERM BP-1239) was inoculated the medium, and then cultured for 6 days at 12°C with reciprocal shaking at 200 rpm. After culturing, the cultured broth was filtered to recover cells. The cells were then completely washed with water and resuspended in 20 ml of 0.05 M phosphate buffer (pH 7.0), and the suspension was subjected to ultrasonication for 15 to 20 minutes to obtain a homogenate, which was then used as an enzyme solution for the following reaction.

The composition of the reaction mixture was as follows.

Composition of reaction mixture
Enzyme solution    0.5 ml
Substrate, i.e., precursor    2 mg
0.6% Tween 20 aqueous solution    0.4 ml
Water    0.1 ml

As the substrates, i.e., precursor for highly unsaturated fatty acids, sodium stearate, sodium oleate, sodium linolate, and sodium linolenate were separately used. Moreover, a control (1) which did not contain substrate, and a control (2) wherein 0.5 ml of 0.05 M phosphate buffer was used in place of the enzyme solution, were prepared. Each reaction was carried out in a test tube (16.5ø × 160 mm) at 28°C for three hours while shaking at 240 rpm. After the reaction, the reaction mixture was cooled to -20°C to terminate the reaction, and the reaction mixture was immediately lyophilized. The lyophilitate was treated with a mixture of absolute methanol and hydrochloric acid (95:5) at 20°C for five hours to convert fatty acids in the reaction mixture to methyl esters thereof, and extracted with 4 ml of ethyl ether to extract the resulting methyl esters. The extracts obtained from the above-mentioned reactions were then subjected to chromatography to determine the composition of methyl esters of the produced fatty acids. The amount of each highly unsaturated fatty acid produced during the present reaction was calculated by subtracting a total of measurements of the control (1) and control (2) from a measurement of the corresponding highly unsaturated fatty acid from each reaction. Table 2 shows the consumption of the substrates and the amounts produced of highly unsaturated fatty acids, i.e., γ-linolenic acid (GLA), bishomo-γ-linolenic acid (BGLA), arachidonic acid (ARA), and eicosapentaenoic

acid (EPA).

## Table 2

| Substrate (sodium salt) | Consumption of substrate (μg) | Production of highly unsaturated fatty acid | | | |
|---|---|---|---|---|---|
| | | GLA (μg) | BGLA (μg) | ARA (μg) | EPA (μg) |
| Stearate | 190 | 0.8 | 0.3 | 4.5 | 0.2 |
| Oleate | 205 | 1.2 | 0.8 | 35 | 8.5 |
| Linolate | 171 | 8.5 | 1.4 | 46 | 36 |
| Linolenate | 120 | 0.7 | 0.5 | 11 | 32 |

Moreover, the above-mentioned mixture of methyl esters of fatty acids was fractionated into fractions each containing an individual fatty acid methyl ester. Each fraction was then evaporated to dryness to obtain a fatty acid methyl ester preparation. The preparation was then analyzed by gas chromatography, high performance liquid chromatography, Mass spectrum analysis, and NMR analysis, in comparison with a conventional preparation. The analytical data of the reaction product and the data of the corresponding conventional preparation were identical for each fatty acid.

Example 2.

Mortierella elongata IFO 8570, Mortierella alpina IFO 8568, Mortierella exigua IFO 8571, Mortierella hygrophila IFO 5941, and Mortierella parvispora IFO 8574 were cultured according to the same procedure as described in Example 1 to obtain cultured cells; which cells were then lyophilized. The lyophilized cells were used to prepare the following reaction mixtures, which were then subjected to reaction.

Composition of reaction mixture
Lyophilized cells    10 mg
0.05 M phosphate buffer (pH 7.0)    0.5 ml
Sodium linolate    2 mg
0.6% Tween aqueous solution    0.4 ml
Water    0.1 ml

The reaction was carried out at 20°C for 10 hours and the reaction mixtures were extracted and analyzed according to the same procedure as described in Example 1.

For each strain tested, the amounts of substrate consumption and the amounts produced of γ-linolenic acid (GLA), bishomo-γ-linolenic acid (BGLA), arachidonic acid (ARA), and eicosapentaenoic acid are set forth in Table 3.

Table 3

| Strains | Consumption of substrate (µg) | Production of highly unsaturated fatty acids | | | |
|---|---|---|---|---|---|
| | | GLA (µg) | BGLA (µg) | ARA (µg) | EPA (µg) |
| IFO 8570 | 241 | 5.2 | 1.4 | 49 | 39 |
| IFO 8568 | 181 | 3.6 | 0.7 | 63 | 29 |
| IFO 8571 | 175 | 2.7 | 0.6 | 35 | 17 |
| IFO 5941 | 221 | 6.3 | 1.1 | 46 | 43 |
| IFO 8574 | 136 | 2.5 | 0.6 | 31 | 13 |

As seen from Table 3, the strains tested converted a substrate linolate to highly unsaturated fatty acids.

**Claims**

1. A process for the production of a mixture of highly unsaturated fatty acids comprising the steps of:
    (1) culturing a microorganism belonging to the genus Mortierella and capable of synthesizing highly unsaturated fatty acids;
    (2) preparing intact cells or products derived from the cells, having an ability to synthesize highly unsaturated fatty acids, from a precursor thereof;
    (3) converting the precursor to the highly unsaturated fatty acids in the presence of intact cells or a product derived from cells to obtain a reaction mixture containing the highly unsaturated fatty acids; and
    (4) recovering a mixture of the highly unsaturated fatty acids from the reaction mixture.

2. A process according to claim 1, wherein the microorganism belonging to the genus Mortierella is selected from the group of Mortierella elongata, Mortierella alpina, Mortierella exigua, Mortierella hygrophila, and Mortierella parvispora.

3. A process according to claim 2, wherein Mortierella elongata is Mortierella elongata SAM 0219 FERM BP-1239.

4. A process for the production of a highly unsaturated fatty acid comprising the steps of:
    (1) culturing a microorganism belonging to the genus Mortierella and capable of synthesizing highly unsaturated fatty acids;
    (2) preparing intact cells or products derived from cells having an ability to synthesize highly unsaturated fatty acids from a precursor thereof;
    (3) converting the precursor to the highly unsaturated fatty acids in the presence of the intact cells or the product derived from cells to obtain a reaction mixture containing the highly unsaturated fatty acids;
    (4) recovering a mixture of the highly unsaturated fatty acids from the reaction mixture; and
    (5) isolating an individual highly unsaturated fatty acid from the mixture.

5. A process according to claim 4, wherein the microorganism belonging to the genus Mortierella is selected from the group of Mortierella elongata, Mortierella alpina, Mortierella exigua, Mortierella hygrophila, and Mortierella parvispora.

6. A process according to claim 5, wherein Mortierella elongata is Mortierella elongata SAM 0219 FERM BP-1239.

7. A process according to any preceding claim wherein the highly unsaturated fatty acid is selected from the group consisting of α-linolenic acid, γ-linolenic acid, bishomo-γ-linolenic acid, arachidonic acid, eicosapentaenoic acid, and docosahexanoic acid.

8. A process according to any preceding claim wherein the product derived from cells is selected from the group consisting of dried cells, cells treated with an organic solvent, disrupted cells, and immobilized products thereof.

9. A process according to any preceding claim, wherein the precursor is selected from the group consisting of fatty acids, salts of fatty acids, esters of fatty acids, and fats or oils containing the fatty acids.

10. A process according to claim 9, wherein the fatty acid is selected from the group consisting of acetic acid, butyric acid, caproic acid, caprylic acid, capric acid, lauric acid, myristic acid, miristoleic acid, palmitic acid, palmitoleic acid, stearic acid, oleic acid, linoleic acid, linolenic acid, octadecatetraenoic acid, arachidic acid, eicosenoic acid, eicosadienoic acid, eicosatrienoic acid, and eicosatetraenoic acid.